# EUROPÄISCHE PATENTANMELDUNG

(11) **EP 0 855 186 A2**
(43) Veröffentlichungstag der Anmeldung: **29.07.1998**
(21) Anmeldenummer: 98250140.5
(22) Anmeldetag: 10.02.1995
(51) Int. Cl.: A61K 49/00

(54) **Gas enthaltende Mikropartikel, deren Verwendung in der Ultra-schalldiagnostik**

(30) Priorität: 23.02.1994 DE 4406474
(62) Teilanmeldung aus: 95909702.3
(71) Anmelder: SCHERING AKTIENGESELLSCHAFT, 13353 Berlin (DE)
(72) Erfinder: Heldmann, Dieter, Dr., 10555 Berlin (DE); Weitschies, Werner, Dr., 10961 Berlin (DE); Fritzsch, Thomas, Dr., 12169 Berlin (DE); Speck, Ulrich, Prof. Dr., 13465 Berlin (DE); Hauff, Peter, Dr., 12627 Berlin (DE)

(57) **Zusammenfassung**

Die Erfindung betrifft neue Mikropartikel, bestehend aus der Mischung einer gesättigten C₁₂-C₂₆-Fettsäure mit Galactose und einer bei Körpertemperatur gasförmigen Komponente, dadurch gekennzeichnet, daß als gasförmige Komponente Hexafluorethan, Dekafluorbutan und/oder Perfluorpropan enthalten ist, diese Mikropartikel enthaltende diagnostische Mittel sowie deren Verwendung in der Ultraschalldiagnostik.

## Beschreibung

Die Erfindung betrifft den in den Patentansprüchen gekennzeichneten Gegenstand, das heißt neue, Gas enthaltende Mikropartikel, diese enthaltende diagnostische Mittel, deren Verwendung in der Ultraschalldiagnostik sowie Verfahren zur Herstellung der Partikel und Mittel.

Seit der Entdeckung durch Gramiak Ende der 60er Jahre, daß Ultraschallkontraste durch Gasbläschen in Flüssigkeiten (Blut) hervorgerufen werden, wurden die verschiedenartigsten Typen gashaltiger Ultraschallkontrastmittel entwickelt und in der Literatur beschrieben.

Der einfachste Typ Ultraschallkontrastmittel läßt sich z.B. durch heftiges Schütteln, durch schnelles Aufziehen und wieder Ausspritzen in (aus) eine(r) Injektionsspritze (sogenanntes "Pumpen") oder Beschallen mit Ultraschall von Lösungen, wie Salzlösungen, Farbstofflösungen oder von vorher entnommenem Blut, erzeugen. Durch die vorbeschriebenen Methoden werden die für die Echokontrastgebung erforderlichen Gasbläschen in das Suspensionsmedium eingebracht. Je nach Wahl des Mediums kann ein mehr oder weniger stabilisierender Effekt des Mediums auf die Mikrogasbläschen erreicht werden.
Derartige Kontrastmittel werden z.B. in der EP 0 077 752 beschrieben. Als Suspensionsmedium finden hier Gemische aus viskositätserhöhender Substanz und Tensid Verwendung. Als Gase werden in der EP 0 077 752 Luft und CO₂ offenbart. Die genannten Kontrastmittel sowie ähnliche, nach den genannten Methoden herstellbare Kontrastmittel, sind mit dem schwerwiegenden Nachteil behaftet, daß die Gasbläschengröße stark variiert und nur schlecht reproduzierbar ist, wodurch ein hohes Embolie-Risiko besteht. Weiterhin lösen sich die durch das Suspensionsmedium nur gering stabilisierten Bläschen schnell auf, so daß ein Kontrasteffekt nur über einen kurzen Zeitraum beobachtet werden kann. Ein Linksherzkontrast nach intravenöser Gabe ist in der Regel bei derartigen Kontrastmittel nicht beobachtbar.

In der WO 93/05819 werden Kontrastmittel auf der Basis von Bläschenemulsionen beschrieben, bei denen jedoch anstelle der herkömmlichen Gase (Luft, Stickstoff, CO₂ und Edelgase), Gase mit einem gewissen Q-Faktor verwendet werden. Bei diesen Gasen handelt es sich in der Regel um halogenierte Kohlenwasserstoffe. Durch die Verwendung dieser konnte der Kontrasteffekt und insbesondere die Signaldauer verlängert werden. Da die Gase auch in diesem Fall - wie schon bei den zuvor beschriebenen Kontrastmitteln - z.B. durch Umpumpen mehrerer Ausgangsstoffe zwischen zwei Spritzen via Dreiwegehahn in das Suspensionsmedium eingebracht werden, zeigen auch diese Mittel eine sehr inhomogene Bläschengrößenverteilung mit dem damit verbundenen Embolie-Risiko.

Die Verwendung bestimmter fluorierter Substanzen als Gase für verschiedene Typen von Ultraschallkontrastmitteln wird auch in der EP 0 554 213 beansprucht. Auch diese Schrift betrifft nicht die erfindungsgemäßen Mikropartikelpräparationen. Das nächstliegende Beispiel (3) der EP 0 554 213 enthält Mikropartikel auf Galactosebasis [wie sie in der EP 0 052 575 / Beispiel 1 beschrieben werden], die anstelle von Luft SF₆ enthalten. Die für diese Partikel beobachteten Effekte sind jedoch schwach und übersteigen die Streuung zwischen den Doppelwerten der Proben nur wenig (siehe Tabelle 3 der EP 0 554 213).

Kontrastmittel mit standardisierter Bläschengröße werden in der EP 0 122 624 und 0 123 235 beschrieben. Diese Kontrastmittel bestehen aus gasenthaltenden Mikropartikeln. Als Partikelmaterial werden Gemische aus grenzflächenaktiven Substanzen wie z.B. Fettsäuren und nicht-grenzflächenaktiven Substanzen wie z.B. Sacchariden verwendet, als Gas wird Luft offenbart. Zwar zeigen diese Mittel die erwünschte Standardisierung bezüglich der Bläschengröße, jedoch lösen sich die Gasbläschen relativ schnell in der Blutflüssigkeit auf, so daß das diagnostische Zeitfenster klein ist.

Ähnliche Kontrastmittel werden in der EP 0 365 467 offenbart. Diese überstehen nach i. v. Applikation des Mittels die Passage des Lungenkapillarbettes und sind somit für die Kontrastierung des linken Herzens und des arteriellen Blutes geeignet. Für einen ausreichend langen und intensiven Kontrasteffekt müssen diese, wie auch die in der EP 0 122 624 und 0 123 235 beschriebenen Kontrastmittel, jedoch in einer Konzentration verabreicht werden, die nicht blutisoton ist, was zu den bekannten Irritationen beim Patienten führen kann.

Weitere Ultraschallkontrastmittel mit standardisierter Bläschengröße werden in der DE 38 03 972 und EP 0 441 468 sowie in der DE 38 03 972 und EP 0 357 163 offenbart. Die beiden erstgenannten Schriften beschreiben Mikropartikel für die Ultraschalldiagnostik bei denen die bildgebenden Substanzen (Gase bzw. niedrig siedende organische Flüssigkeiten) in gekapselter Form vorliegen. Als Hüllmaterial finden Polycyanacrylate (DE 38 03 972) bzw. polymerisierte Aldehyde (EP 0 441 468) Anwendung. Die letztgenannten Schriften beschreiben Mikropartikel bei denen die Gase (bzw. niedrig siedende organische Flüssigkeiten) in komplexierter Form (d.h. in Form eines Wirt/Gast-Komplexes) vorliegen. Obgleich bei diesen Schriften als Gase bzw. niedrig siedende Flüssigkeiten neben den gebräuchlichen Stoffen wie Luft, Stickstoff, auch halogenierte Kohlenwasserstoffe (wie z.B. Brommethan oder Dibromdifluormethan) oder im Falle EP 0 357 163 und EP 0 441 468 auch Schwefelhexafluorid offenbart werden, wird für aus diesen Mikropartikeln bereitete Ultraschallkontrastmittelpräparationen kein bzw. ein nur geringfügiger Einfluß der eingeschlossenen bildgebenden Komponente auf die Kontrastintensität bzw. -dauer beobachtet bzw. beschrieben.

Aufgabe der vorliegenden Erfindung war es daher, ein Kontrastmittel mit definierter Bläschengröße für die Ultraschalldiagnostik bereitzustellen, das in der Lage ist, nach intravenöser Applikation langanhaltende Kontrasteffekte im Blut zu erzielen und dessen Strömungsverhältnisse auf der rechten und linken Herzseite für Ultraschall sichtbar zu machen. Insbesondere sollte durch das Kontrastmittel eine diagnostisch auswertbare Kontrastierung bereits in einem Dosisbereich erreicht werden können, in welchem das Mittel weitgehend blutisoton ist.

Darüber hinaus sollten auch die übrigen an ein in-vivo Kontrastmittel zu stellenden Anforderungen erfüllt sein. Neben einer guten Verträglichkeit ist für ein modernes Kontrastmittel insbesondere ein breites Anwendungsspektrum erwünscht, so sollte es auch für die Darstellung der Durchblutung anderer Organe bzw. Gewebe wie z.B. Myokard, Leber, Milz, Niere und Gehirn, oder nach peroraler oder rektaler Applikation auch für die Darstellung des Gastrointestinaltraktes geeignet sein. Nach Gabe in die Harnblase sollte eine Darstellung des Harnflusses ebenso möglich sein, wie eine Kontrastierung der Tuben nach intrauteriner Applikation. Auch sollte das Kontrastmittel universell in den verschiedenen sonographischen Modi (z.B. B-Mode, Doppler, "Harmonic Imaging") einstetzbar sein.

Diese Aufgabe wird durch die vorliegende Erfindung gelöst.

Es wurde gefunden, daß Mikropartikel bestehend aus der Mischung einer gesättigten C₁₂-C₂₆-Fettsäure mit Galactose und einer bei Körpertemperatur gasförmigen Komponente, dadurch gekennzeichnet, daß als gasförmige Komponente Hexafluorethan, Dekafluorbutan und/oder Perfluorpropan enthalten ist, das gestellte Anforderungsprofil erfüllen und daher hervorragend als Kontrastmittel in der Ultraschalldiagnostik geeignet sind.

Die durch Suspendieren der erfindungsgemäßen Mikropartikel in einem flüssigen Träger erhaltenen Ultraschallkontrastmittel führen insbesondere zu einer - im Vergleich zum bekannten Stand der Technik - überraschend intensiven und langanhaltenden Kontrastierung. Dadurch kann die für eine Bildgebung erforderliche Dosis gegenüber den in der EP 0 365 467 offenbarten Kontrastmittel um 90% und mehr reduziert werden (siehe auch Beispiel 9). Auf diese Weise können Kontrastmittel erhalten werden, die blutisoton oder nahezu blutisoton sind, wodurch die Verträglichkeit deutlich erhöht wird. Aufgrund der langanhaltenden Kontrasteffekte eignen sich die erfindungsgemäßen Mittel insbesondere auch als "blood pool agents".

Die erfindungsgemäßen Mikropartikel enthalten die nicht-grenzflächenaktiven Feststoffe in einer Konzentration von mindestens 90, vorzugsweise in einer Konzentration von 95 Gewichtsprozent.

Den genannten fluorierten Gasen können gewünschtenfalls auch bis zu isomolare Mengen an Stickstoff beigemischt werden.

Die erfindungsgemäßen Partikel sind auf vielfältige Weise herstellbar. Sie können z.B. erhalten werden, indem die in der EP 0 365 467, offenbarten Partikel mit den zuvor genannten Gasen behandelt werden, um einen Austausch des in den Partikeln enthaltenen Gases (in der Regel Luft) gegen die gewünschten halogenierten Verbindungen zu erreichen. Dieser Gasaustausch geschieht vorteilhafterweise, indem die jeweiligen Partikel in ein entsprechendes Gefäß eingebracht werden, welches anschließend evakuiert und nachfolgend mit dem gewünschten Gas belüftet wird. Die Inkubation kann auch direkt in den Vials, in denen die Partikel zum Anwender gelangen, erfolgen. Der Gasaustausch kann im Prinzip auch an jeder anderen Stelle des Herstellungsprozesses erfolgen. So besteht insbesondere bei den Partikeln der EP 0 365 467, die Möglichkeit, das Gas während des Zerkleinerungsprozesses, d.h. bei Mahlung z.B. in einer mit dem gewünschten Gas betriebenen "Luftstrahlmühle", einzubringen. Die Atmosphäre des gewünschten Gases ist vorzugsweise bei den weiteren Herstellungsschritten beizubehalten.

Neben dem zuvor beschriebenen nachträglich durchgeführten Gasaustausch kann das Gas aber auch bereits während der Partikelherstellung eingebracht werden. Dabei wird vorteilhafterweise analog zu den in der EP 0 365 467, beschriebenen Methoden verfahren, wobei sämtliche Reaktionslösungen zuvor mit dem gewünschten halogenierten Gas gesättigt werden und die gesamte Herstellung unter einer Atmosphäre des gewünschten, halogenierten Gases durchgeführt wird.

Eine Variante des vorbeschriebenen Verfahrens besteht darin, daß zunächst lediglich die nicht-grenzflächenaktive Substanz unter sterilen Bedingungen aus einer mit dem gewünschten Gas gesättigten Lösung rekristallisiert wird. Anschließend wird die grenzflächenaktive Substanz zusammen mit dem nicht-grenzflächenaktiven Feststoff unter sterilen Bedingungen unter der Atmosphäre des gewünschten halogenierten Gases vermischt (agglomeriert) und zerkleinert, bis die gewünschte Partikelgröße von < 10 µm, vorzugsweise < 8 µm, insbesondere 1 - 3 µm erreicht ist. Die Partikelgröße wird in geeigneten Meßgeräten bestimmt.

Ein alternatives Verfahren zur Herstellung der erfindungsgemäßen Mikropartikel besteht darin, die in der EP 0 365 467 beschriebenen Partikel in einem geeigneten, mit dem gewünschten halogenierten Gas gesättigten Medium zu lösen und aus diesem zu rekristallisieren. Trocknung, Zerkleinerung, Abfüllung usw. erfolgen wie zuvor beschrieben, wobei sämtliche nachgeschalteten Herstellungsschritte vorteilhafterweise unter einer Atmosphäre des jeweiligen Gases durchgeführt werden.

Aus den erfindungsgemäßen Mikropartikeln lassen sich, durch Suspendieren der Partikel in einem geeigneten physiologisch verträglichem Medium, leicht die erfindungsgemäßen Mittel herstellen. Das Suspendieren erfolgt - insbesondere bei den löslichen Partikeln - vorteilhafterweise erst unmittelbar vor der Injektion durch den behandelnden Arzt, indem aus einem ersten Behälter das Suspensionsmedium unter sterilen Bedingungen, z.B. mittels einer Spritze entnommen, zu den in einem zweiten Behälter befindlichen Mikropartikeln gegeben wird und anschließend durch kurzes (5 bis 10 Sekunden) kräftiges Schütteln der vereinigten Komponenten eine homogene Suspension hergestellt wird. Die erfindungsgemäßen Mittel werden unmittelbar nach ihrer Herstellung, spätestens jedoch innerhalb von 5 Minuten, entweder als Bolus in eine periphere Vene oder in einen zuvor plazierten Katheter, injiziert.

Die Erfindung betrifft daher auch ein Kit für die Herstellung eines Mikropartikel und Gas enthaltenden Ultraschallkontrastmittels bestehend aus einem ersten Behälter, versehen mit einem Verschluß, der die Entnahme des Inhalts unter sterilen Bedingungen ermöglicht und mit dem flüssigen Suspensionsmedium gefüllt ist und einem zweiten Behälter, versehen mit einem Verschluß, der die Zugabe des Suspensionsmediums unter sterilen Bedingungen ermöglicht, gefüllt mit den erfindungsgemäßen Mikropartikeln und einem Gas oder Gasgemisch welches identisch mit dem in den Mikropartikeln enthaltenen Gas ist, wobei das Volumen des zweiten Behälters so bemessen ist, daß das Suspensionsmedium des ersten Behälters vollständig im zweiten Behälter Platz findet.
Anstelle zweier separater Behälter kann selbstverständlich auch eine vorgefüllte Spritze bestehend aus zwei Kammern, deren eine Kammer das Suspensionsmedium und deren zweite Kammer die Partikel enthält, verwendet werden.

Als physiologisch verträgliche Suspensionsmedien kommen in Frage Wasser, wäßrige Lösungen eines oder mehrerer anorganischer Salze wie physiologische Kochsalzlösung und Pufferlösungen, wäßrige Lösungen von Mono- oder Disacchariden wie Galactose; Glucose oder Lactose, oder Cyclodextrine, ein- oder mehrwertigen Alkoholen, soweit sie physiologisch verträglich sind, z.B. Ethanol, Polyethylenglykol, Ethylenglykol, Glycerin, Propylenglykol, Propylenglykolmethyl-ester. Bevorzugt sind Wasser und physiologische Elektrolytlösungen wie z.B. physiologische Kochsalzlösung sowie wäßrige Lösungen von Galactose und Glucose. Die Konzentration der gelösten Stoffe beträgt 0,1 bis 30 Gewichtsprozent, vorzugsweise 0,5 bis 25 Gewichtsprozent.

Selbstverständlich können den Mitteln unterschiedliche pharmazeutische Hilfsstoffe und Stabilisatoren zugesetzt werden. Auch sind die angegebenen Maße und Prozentwerte als Richtgrößen gedacht. Deren Über- oder Unterschreitung kann im Einzelfall möglich und nützlich sein.

Die erfindungsgemäßen Mittel enthalten je nach Verwendung 5 mg bis 500 mg Partikel pro ml Suspensionsmedium. Die für die Kontrastgebung benötigten Gasbläschen werden durch die Mikropartikel bereitgestellt. Diese sind teilweise an der Oberfläche der Mikropartikel adsorbiert bzw. in den Hohlräumen zwischen den Mikropartikeln oder interkristallin eingeschlossen.

Für intravenöse Anwendungen finden ausschließlich Mittel auf Basis löslicher Partikel Anwendung, für perorale oder rektale Anwendung können darüber hinaus auch Mittel auf Basis unlöslicher Partikel verwendet werden. In Abhängigkeit von der Verwendung variiert auch die jeweils verabreichte Dosis, so werden bei intravenöser Verabreichung in der Regel 0,01 ml bis 1 ml/kg Körpergewicht, bei peroraler Gabe 1 bis 30 ml/kg Körpergewicht appliziert.

Die erfindungsgemäßen Ultraschallkontrastmittel erreichen nach intravenöser Gabe die linke Herzseite und sind somit auch zur Kontrastierung von anderen von der Aorta aus mit Blut versorgten Organen, wie Myokard, Leber, Milz, Niere u.a.m. hervorragend geeignet. Es versteht sich von selbst, daß die erfindungsgemäßen Ultraschallkontrastmittel auch zur Kontrastierung des rechten Herzens und anderer Organe und Körperregionen geeignet sind.

Ein völlig überraschender Vorteil der erfindungsgemäßen Mittel liegt in der Möglichkeit einer außerordentlich starken Dosisreduktion im Vergleich zu den Mitteln des Standes der Technik, wodurch es möglich ist, die Substanzbelastung, die zu applizierenden Volumina bzw. die Osmolarität der Mittel zu senken. Damit können auch Ultraschallkontrastmittel zur Verfügung gestellt werden, die blutisoton oder nahezu blutisoton sind. Ein weiterer Vorteil besteht in der hohen Resistenz gegenüber den eingestrahlten Ultraschallwellen, was zu einer erheblich verbesserten intravitalen Stabilität führt.

Die nachfolgenden Beispiele dienen der näheren Erläuterung des Erfindungsgegenstandes, ohne ihn auf diese beschränken zu wollen.

### Beispiel 1

Mikropartikel hergestellt nach Beispiel 1 der EP 0 365 467 werden in 20 ml Vials zu 2 g abgefüllt. Die Abfüllung erfolgt unter einer Atmosphäre aus Hexafluorethan, welches ebenfalls in den Vials enthalten ist.

### Beispiel 2

Mikropartikel hergestellt nach Beispiel 1 der EP 0 365 467 werden in 20 ml Vials zu 2 g abgefüllt. Die Abfüllung erfolgt unter einer Atmosphäre aus Dekafluorbutan, welches ebenfalls in den Vials enthalten ist.

Perfluorpropan-haltige Partikel können analog zu Beispiel 2 erhalten werden, indem die Abfüllung der Vials unter einer Atmosphäre aus Parfluorbutan erfolgt.

### Beispiel 3

Mikropartikel hergestellt nach Beispiel 1 der EP 0 365 467 werden in 20 ml Vials zu 3 g abgefüllt. Die Abfüllung erfolgt unter einer Atmosphäre aus Hexafluorethan, welches ebenfalls in den Vials enthalten ist.

### Beispiel 4

Mikropartikel hergestellt nach Beispiel 1 der EP 0 365 467 werden in geöffneten Vials in einem evakuierbaren Behältnis gelagert und darin bis auf einen Druck von 50 mbar evakuiert. Danach wird das Behältnis mit Hexafluorethan belüftet, die Vials werden unter einer Hexafluorethanatmosphäre verschlossen.

### Beispiel 5

Es wird analog zu Beispiel 11 verfahren, wobei als Gas anstelle von Hexafluorethan Dekafluorbutan eingesetzt wird.

### Beispiel 6

Es wird analog zu Beispiel 11 verfahren, wobei als Gas anstelle von Hexafluorethan Perfluorpentan eingesetzt wird.

### Beispiel 7

1997 g Galactose werden in 1080 g Wasser gelöst und auf 5 °C abgekühlt. Zu der entstandenen Suspension werden 3 g Lignocerinsäure, die zuvor in 120 g Ethanol gelöst wurde, unter Rühren zugestetzt. Die Suspension wird anschließend bei 40 °C und einem Unterdruck von 50 mbar getrocknet. Das entstandene Produkt wird mit einer Luftstrahlmühle auf eine Partikelgröße von d_{(99%)} < 8 µm zerkleinert. Die Mikropartikel werden zu einem Granulat agglomeriert und in Portionen zu 2 g je 20 ml Vial abgefüllt, evakuiert, mit Dekafluorbutan begast, 24 Stunden lang in der Dekafluorbutanatmosphäre inkubiert und anschließend verschlossen.

### Beispiel 8 (in-vivo Vergleichsbeispiel)

A) 1 g der erfindungsgemäßen Mikropartikel hergestellt nach Beispiel 3 wurde in 2,7 ml Aqua p.i. suspendiert. 2 ml der frisch zubereiteten Suspension wurden einem narkotisierten Beagle-Hund (10 kg Körpergewicht) intravenös injiziert und mit einem Ultraschallgerät das Herz untersucht. Der Kontrastverlauf im linken Ventrikel wurde videodensitometrisch aufgezeichnet und ausgewertet.
B) 1 g Mikropartikel hergestellt nach Beispiel 1 der EP 0 365 467 wurde in 2,7 ml Aqua p.i. suspendiert. 2 ml der frisch zubereiteten Suspension wurden einem narkotisierten Beagle-Hund (10 kg Körpergewicht) intravenös injiziert und mit einem Ultraschallgerät das Herz untersucht. Der Kontrastverlauf im linken Ventrikel wurde videodensitometrisch aufgezeichnet und ausgewertet. Alle übrigen Untersuchungsparameter blieben gegenüber Versuch A) unverändert.

Ergebnis: Die Injektion der erfindungsgemäßen Kontrastmittelpräparation führt zu deutlich intensiverem und deutlich länger anhaltendem Kontrast ("blood pool agent").

### Beispiel 9 (in vivo Vergleichsversuch)

Zu Vergleichszwecken werden Kontrastmittelpräparationen aus
A) Mikropartikeln hergestellt nach Beispiel 1 der EP 0 365 467 und
B) erfindungsgemäßen Mikropartikeln hergestellt nach Beispiel 5 frisch zubereitet. Als Suspensionsmedium wird Wasser p.i. verwendet.
1 ml der jeweiligen Suspensionen werden unmittelbar nach der Zubereitung einem narkotisierten Beagle-Hund (10 kg Körpergewicht) intravenös injiziert und mit einem Ultraschallgerät das Herz untersucht. Der Verlauf der Ultraschallkontrastverstärkung im linken Ventrikel des Herzens wird videodensitometrisch aufgezeichnet und ausgewertet. Die beobachteten Daten (Mittelwerte aus jeweils 3 Versuchen) sind in der nachfolgenden Tabelle zusammengestellt.

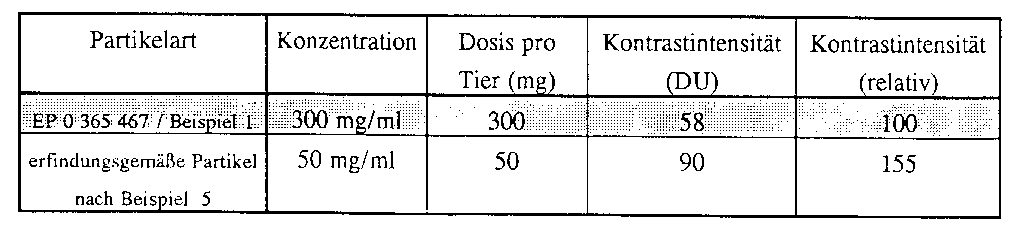

Ergebnis: Die erfindungsgemäßen Kontrastmittel zeigen selbst bei einer Dosisreduktion um den Faktor 6 noch einen intensiveren Kontrast als die Mittel der EP 0 365 467. Von großem Vorteil ist die Blutisotonie der erfindungsgemäßen Präparation im Vergleich zur hypertonen Präparation nach Beispiel 1 der EP 0 365 467. Von großem Vorteil ist die Blutisotonie der erfindungsgemäßen Präparation im Vergleich zu der hypertonen Präparation nach Beispiel 1 der EP 0 365 467.

### Beispiel 10 (in vivo Vergleichsversuch)

Weitere Daten aus einem analog zu Beispiel 9durchgeführter Vergleichsversuch sind in nachfolgender Tabelle zusammengefaßt.

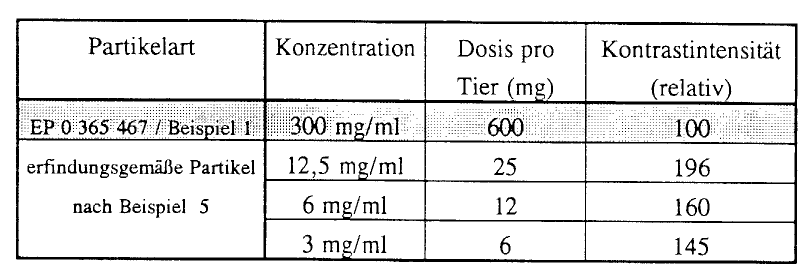

Auch in diesem Fall kann bei Verwendung der erfindungsgemäßen Kontrastmittelpräparation trotz einer Dosisreduktion um bis zum Faktor 100 ein intensiverer Kontrast als bei Verwendung der Kontrastmittelpräparation nach Beispiel 1 der EP 0 365 467 beobachtet werden.

## Patentansprüche

1. Zubereitungen zur Herstellung eines Präparates für die Ultraschalldiagnostik, enthaltend eine bei Körpertemperatur gasförmigen Komponente und Mikropartikel bestehend aus der Mischung einer gesättigten C₁₂-C₂₆Fettsäure mit Galactose und einer bei Körpertemperatur gasförmigen Komponente, dadurch gekennzeichnet, daß als gasförmige Komponente Hexafluorethan, Dekafluorbutan und/oder Perfluorpropan enthalten ist.

2. Mikropartikel nach Anspruch 1, dadurch gekennzeichnet, daß die Galactose in einer Konzentration von 90 - 99,999 Gewichtsprozent enthalten ist.

3. Ultraschallkontrastmittel enthaltend Mikropartikel nach einem der Ansprüche 1 bis 2 in einem physiologisch verträglichen flüssigen Suspensionsmedium gegebenenfalls mit den in der pharmazeutischen Technologie üblichen Zusätzen.

4. Ultraschallkontrastmittel nach Anspruch 3 enthaltend als physiologisch verträgliches Suspensionsmedium Wasser, physiologische Elektrolytlösung, eine wäßrige Lösung von ein- oder mehrwertigen Alkoholen wie Glycerin, Polyethylenglycol oder von Propylenglykolmethylester oder eine wäßrige Lösung eines Mono- oder Disaccharides.

5. Ein Kit für die Herstellung eines Mikropartikel und Gas enthaltenenden Ultraschallkontrastmittels bestehend aus
a) einem ersten Behälter, versehen mit einem Verschluß, der die Entnahme des Inhalts unter sterilen Bedingungen ermöglicht und mit dem flüssigen Suspensionsmedium gefüllt ist und
b) einem zweiten Behälter versehen mit einem Verschluß, der die Zugabe des Suspensionsmediums unter sterilen Bedingungen ermöglicht, gefüllt mit Mikropartikeln nach einem der Ansprüche 1 bis 4 und einem Gas oder Gasgemisch welches identisch mit dem in den Mikropartikeln enthaltenen Gas ist, wobei das Volumen des zweiten Behälters so bemessen ist, daß das Suspensionsmedium des ersten Behälters vollständig im zweiten Behälter Platz findet.
